# EUROPEAN PATENT SPECIFICATION

(11) **EP 0 686 052 B1**
(45) Date of publication and mention of the grant of the patent: **03.07.2002**
(21) Application number: 94908558.3
(22) Date of filing: 24.02.1994
(51) Int. Cl.: A61M 25/00

(54) **A CATHETER OF ELECTRICALLY CONDUCTIVE SYNTHETIC MATERIAL**
AUS ELEKTRISCH KONDUKTIVEN, SYNTHETISCHEN MATERIALIEN BESTEHENDER KATHETER
CATHETER COMPOSE D'UN MATERIAU SYNTHETIQUE ELECTROCONDUCTEUR

(30) Priority: 26.02.1993 SE 9300655
(43) Date of publication of application: 13.12.1995
(73) Proprietor: Nilsson, Leif, 260 40 Viken (SE)
(72) Inventor: Nilsson, Leif, 260 40 Viken (SE)
(74) Representative: Norén, Per Bo Arne
(86) International application number: PCT/SE94/00154
(87) International publication number: WO 94/19046

(56) References cited:
- WO-A-86/05103
- WO-A-91/00074
- WO-A-91/00118
- US-A- 5 007 437
- Radio & Television, No. 1, 1980 (Specialtidningsfoerlaget AB, Stockholm, Sweden), JOERGEN GUNDERSEN: "Funktionsrubbningar i urinblasan avhjaelpta med elektrisk stimulans", page 38.
- Medicinsk Teknik, No. 6, 1986 (Ingenjoersfoerlaget AB, Stockholm, Sweden), SVEN ERIK JENSEN: "Mindre problem med hal kateter", pages 29-30.

## Description

The present invention relates to a catheter for insertion into a patient's urethra, which comprises an elongated open-ended hollow body of circular cross-section having proximal and distal ends, wherein the catheter body has provided along its length one or more resilient annular projections which project radially outwards and are intended to contact the inner walls of the urethra, the catheter body and each projection are electrically conductive and the proximal end of the catheter is capable of being connected to a low voltage source; and wherein at least one of the projections comprises a plate-like bulge permanently fixed to the proximal end of the catheter.

### Technical background

SE-A-465 999 and corresponding WO-publication 91/00074 disclose an incontinence system including a catheter tube to be inserted in the urethra. Said catheter tube is provided with a first fixed end collar, an intermediate fixed collar with a smaller diameter located upstreams said first collar and a third fixed collar located upstreams said second collar. All three collars project radially outwards from the catheter body.

US-A-5 007 487 discloses a catheter for insertion into the urethra and having at least one swelling surrounding the catheter body and projecting radially outwards therefrom. Electrically conductive material is included in the catheter body and the swelling.

### Summary of the invention

The present invention is directed to an improved catheter of the kind described and the purpose is to provide the catheter body with means for selectively limiting the extent to which the catheter body can be inserted. This is achieved according to the characterizing part of Claim 1.

Other advantages and features of the invention will be apparent from the remaining claims and also from the following description of an exemplifying embodiment of the invention, which is made with reference to the accompanying drawing, in which
Fig. 1 is a longitudinal sectional view of a first embodiment of the improved catheter; and
Fig. 2 illustrates a second exemplifying embodiment of the inventive catheter.

The inventive catheter includes an elongated or relatively elongated hollow catheter body 10 which is made of an electrically conductive and resilient synthetic material, or from a resilient synthetic material which has been made electrically conductive in manufacture by incorporating electrically conductive material thereinto, such as carbon black, carbon fibres and the like. The catheter body will preferably have a length suitable for use by both men and women.

The illustrated catheter body 10 has an integral platelike bulge 11 at the proximal end of the catheter. The illustrated bulge 11 has a dish-shape and extends circumferentially around said body. The part of the dish-shaped bulge 11 which extends in the axial direction of the catheter body has a length which is greater than the outer diameter of said body. This bulge 11 is comprised of or includes an electrically conductive material.

The catheter also includes an annular bulge 13, as illustrated. This bulge 13 can be displaced axially along the catheter body 10. In this case, the bulge 13 is preferably capable of being press-fitted onto the catheter body 10, so as to enable said bulge to be held in selected positions along said body. It will be understood that the bulge 13 which is intended for displacement along the catheter body will not be electrically conductive and is intended to provide means for limiting the extent to which the catheter body 10 can be inserted.

The illustrated exemplifying embodiment of the inventive catheter also includes at least one further, preferably dish-shaped bulge 12 which is spaced axially from the first thickening 11 and is formed integrally with the catheter body 10. This second bulge 12 also has electrically conductive properties preferably of a smaller size than the bulge 11.

In the illustrated embodiments, the respective forwardly located bulges 11, 12 include "wings" which are angled rearwardly with regard to the insertion direction, while the rearward bulge 13 is provided with "wings" which are angled in the opposite direction.

As before mentioned, a catheter of the aforedescribed kind can be easily connected to a low a.c. voltage source (not shown), and the applied voltage may be pulsating or constant, or even of the microwave kind. The current source can be connected to one end of the catheter with the aid of conventional battery terminal clamps.

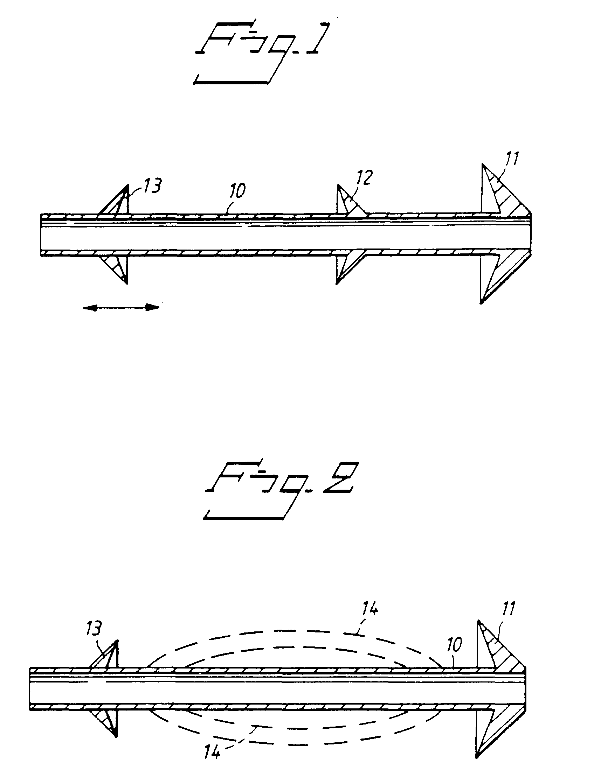

The aforedescribed bulges 11, 12 on the catheter body 10 may also be combined with or optionally replaced by a known inflatable bladder, as indicated in broken lines in Fig. 2 and referenced 14. Such a bladder 14 may have a varying axial extension.

## Claims

1. A catheter for insertion into a patient's urethra, which comprises an elongated open-ended hollow body (10) of circular cross-section having proximal and distal ends, wherein the catheter body (10) has provided along its length one or more resilient annular projections (11; 12; 13; 14) which project radially outwards and are intended to contact the inner walls of the urethra, wherein at least one of the projections comprises a plate-like bulge (11) permanently fixed to the proximal end of the catheter, the catheter further comprising a further annular bulge (13) fitted to the catheter body (10) which is made of an electrically non-conductive material, **characterized in that** the further annular bulge (13) is movable along the length of the catheter and the catheter body and each other projection is made of an electrically conductive material, the proximal end of the catheter being capable of being connected to a low voltage source.

2. A catheter according to Claim 1, **characterized in that** one of the projections has the form of an inflatable bladder (14) which extends between the resilient annular projection fixed to the proximal end of the catheter comprising the plate-like bulge (11) and the further annular bulge (13) which is movable along the length of the catheter body (10).

3. A catheter according to Claim 1, **characterized in that** the electrical material is selected from the group consisting of carbon black and carbon fiber.

## Patentansprüche

1. Katheter zum Einsetzen in die Harnröhre eines Patienten, mit einem langgestreckten, offene Enden aufweisenden hohlen Körper (10) von kreisförmigen Querschnitt, der proximale und distale Enden aufweist, wobei der Katheterkörper (10) entlang seiner Erstreckung eine oder mehrere elastische ringförmige Vorsprünge (11; 12; 13; 14) aufweist, welche radial auswärts vorstehen und dazu dienen, in Kontakt mit den inneren Wänden der Harnröhre zu treten, wobei zumindestens einer der Vorsprünge eine plattenartige Ausbauchung (11) aufweist, die permanent am proximalen Ende des Katheters fixiert ist, wobei der Katheter ferner eine weitere ringförmige Ausbauchung (13) aufweist, die am Katheterkörper (10) angeordnet ist, die aus einem elektrisch nicht leitfähigen Material besteht, **dadurch gekennzeichnet, dass** die weitere ringförmige Ausbauchung (13) beweglich entlang der Längserstreckung des Katheters ist und der Katheterkörper und jeder weitere Vorsprung aus einem elektrisch leitfähigen Material besteht, wobei das proximale Ende des Katheters an eine Niederspannungsquelle anschließbar ist.

2. Katheter nach Anspruch 1, **dadurch gekennzeichnet, dass** einer der Vorsprünge die Form eines inflatierbaren Balgs (14) aufweist, der sich zwischen dem elastischen ringförmigen Vorsprung, der am proximalen Ende des Katheters angeordnet ist und die plattenartige Ausbauchung (11) aufweist, und der weiteren ringförmigen Ausbauchung (13) erstreckt, die entlang der Länge des Katheterkörpers (10) beweglich ist.

3. Katheter nach Anspruch 1, **dadurch gekennzeichnet, dass** das elektrische Material aus der Gruppe bestehend aus Ruß und Carbonfasern ausgewählt ist.

## Revendications

1. Un cathéter pour insertion dans l'urètre d'un patient qui comporte un corps creux (10) ouvert à son extrémité avec une section circulaire ayant une extrémité proximale et une extrémité distale, dans lequel le corps du cathéter (10) comporte sur sa longueur une ou plusieurs saillies annulaires élastiques (11, 12; 13, 14) qui se projettent radialement vers l'extérieur et sont prévus pour entrer en contact avec les parois internes de l'urètre, dans lequel au moins une des saillies comporte un renflement à structure stratifiée (11) fixé en permanence à l'extrémité proximale du cathéter, le cathéter comportant en outre un renflement annulaire supplémentaire (13) adapté au corps du cathéter (10) et réalisé dans un matériau isolant, **caractérisé en ce que** le renflement annulaire supplémentaire (13) peut être déplacé le long du cathéter et **en ce que** le corps du cathéter et toute autre saillie sont réalisés en matériau conducteur, l'extrémité proximale pouvant être connectée à une source de basse tension.

2. Un cathéter selon la revendication 1, **caractérisé en ce qu'**une des saillies a la forme d'une vessie gonflable (14) qui s'étend entre la saillie annulaire élastique fixée à l'extrémité proximale du cathéter comportant le renflement à structure stratifiée (11) et le renflement annulaire supplémentaire (13) qui peut être déplacé le long du corps du cathéter (10).

3. Un cathéter selon la revendication 2, **caractérisé en ce que** le matériau conducteur est sélectionné dans un groupe constitué du noir de carbone et de la fibre de carbone.
